(19) **Europäisches Patentamt European Patent Office Office européen des brevets**

(11) **EP 3 081 628 B1**

(12) **EUROPEAN PATENT SPECIFICATION**

(45) Date of publication and mention of the grant of the patent:
**09.12.2020 Bulletin 2020/50**

(51) Int Cl.:
***C12M 1/00*** *(2006.01)*  ***B01F 13/00*** *(2006.01)*

(21) Application number: **14870351.5**

(86) International application number:
**PCT/JP2014/082512**

(22) Date of filing: **09.12.2014**

(87) International publication number:
**WO 2015/087858 (18.06.2015 Gazette 2015/24)**

(54) **ALGAE CULTURING APPARATUS AND ALGAE CULTURING SYSTEM**

ALGENKULTIVIERUNGSVORRICHTUNG UND ALGENKULTIVIERUNGSSYSTEM

APPAREIL DÉDIÉ À LA CULTURE D'ALGUES ET SYSTÈME DE CULTURE D'ALGUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2013 JP 2013254530**

(43) Date of publication of application:
**19.10.2016 Bulletin 2016/42**

(73) Proprietor: **Electric Power Development Co., Ltd. Tokyo 104-8165 (JP)**

(72) Inventor: **MATSUMOTO Mitsufumi
Kitakyusyu-shi
Fukuoka 808-0111 (JP)**

(74) Representative: **Lavoix
Bayerstrasse 83
80335 München (DE)**

(56) References cited:
**WO-A1-2010/012028   WO-A1-2012/077081
JP-A- H07 176        JP-A- H1 066 561
JP-A- H05 111 376    JP-A- H07 250 668
JP-A- 2004 057 967   JP-A- 2006 035 021
JP-A- 2011 254 724   JP-A- 2012 175 964
JP-A- 2013 085 534   US-A1- 2013 102 076**

## Description

Technical Field

The present invention relates to an algae culturing apparatus for culturing photosynthesizing algae which perform photosynthesis by absorbing light and taking carbon dioxide hereinto, and an algae culturing system including the algae culturing apparatus.

Background Art

WO 2012/077081 A1 discloses a fluid agitator device for facilitating development of algae or micro-algae in trays or photobioreactors.

US 2013/102076 A1 discloses methods and apparatus for promoting the growth of an aquatic photosynthetic organism within a growth medium in a photobioreactor using a luminescent material targeting the aquatic photosynthetic organism in the photobioreactor.

JP 2011-254724 A discloses a photosynthetic microalgae culture apparatus for surely preventing sedimentation and deposition of photosynthetic microalgae.

JP H07-000176 A discloses a device for culturing photosynthetic microorganism where the microorganism is prevented from adhering to the light irradiation face with light utilization efficiency increased.

In recent years, as microorganisms for absorbing and fixing carbon dioxide which is a main factor of global warming by photosynthetic reaction, photosynthetic microalgae has attracted attention.

The photosynthetic microalgae are cultured by a culturing apparatus having a culture tank or a culture pond in which a culture solution is accommodated. In the culturing apparatus, in order to prevent decay due to sedimentation or accumulation of cultured photosynthetic microalgae and increase productivity, it is necessary to stir a liquid mixture of photosynthetic microalgae and a culture solution (for example, refer to patent document 1).

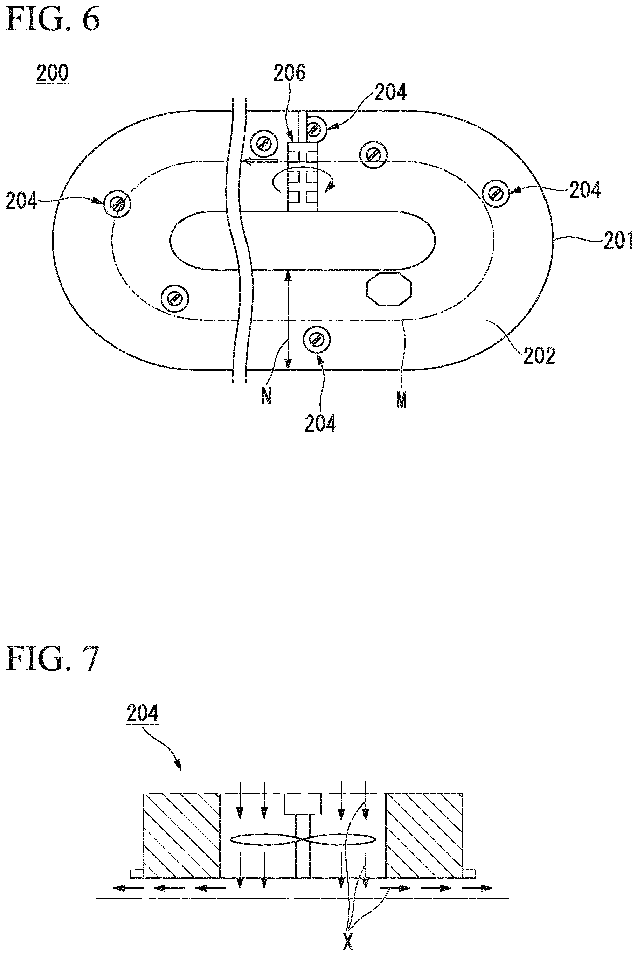

FIG. 6 is a plan view showing a schematic configuration of an algae culturing apparatus disclosed in patent document 1. FIG. 7 is a sectional view showing a schematic configuration of a stirring device shown in FIG. 6. In FIG. 6, M indicates a center line (hereinafter, referred to as a "center line M") of a culture tank 201, and N indicates a width (hereinafter, referred to as a "width N") of the culture tank 201.

Referring to FIGS. 6 and 7, patent document 1 discloses an algae culturing apparatus 200 which includes the culture tank 201, a plurality of stirring devices 204, and moving device 206. The culture tank 201 accommodates the liquid mixture of the photosynthetic microalgae and the culture solution, and cultures the photosynthetic microalgae. In a state where each of the stirring devices 204 is positioned at a position separated by a constant height upward from a bottom portion of the culture tank 201 in the liquid mixture 202 of the culture tank 201, the stirring device 204 sucks the liquid mixture 202 and discharges the liquid mixture 202 toward the bottom portion to stir the photosynthetic microalgae of the bottom portion. The moving device 206 moves the stirring devices 204 into the liquid mixture 202. In the stirring device 204, the flowing liquid mixture moves in an arrow X direction.

By using the algae culturing apparatus 200 configured as described above, it is possible to prevent decay due to sedimentation or accumulation of the cultured photosynthetic microalgae to increase productivity.

Citation List

Patent Document

Patent Document 1: Japanese Unexamined Patent Application, First Publication No. 2011-254724

Summary of Invention

Technical Problem

However, in a case where microalgae are cultured using the algae culturing apparatus 200 disclosed in patent document 1, in order to drive the stirring devices 204 and the moving device 206, much power is required.

That is, there is a problem that much power is required to culture a predetermined amount (for example, 1 kg) of microalgae.

The inventors measured power required for the culture of microalgae of 1 kg using a commercial raceway type algae culturing system and a commercial column type algae culturing system for two years, and found that $74.4 \pm 28.2$ (kWh/kg) was used in the raceway type algae culturing system, and $223.2 \pm 38.0$ (kWh/kg) was used in the column type algae culturing system.

From the above-described values, in the related art, it is demonstrated that much power is required to culture a predetermined amount (for example, 1 kg) of microalgae.

Accordingly, an object of the present invention is to provide an algae culturing apparatus and an algae culturing system capable of preventing decay due to sedimentation or accumulation of algae and culturing a predetermined amount of algae with less power than that of a culturing apparatus in the related art.

Solution to Problem

An aspect of the present invention is an algae culturing apparatus according to claim 1.

Preferred embodiments are detailed in the dependent claims.

Other aspect of the present invention is an algae culturing system according to claim 16.

Preferred embodiments are detailed in the dependent claims.

Advantageous Effects of Invention

According to the algae culturing apparatus of the present invention, since the flow generator moves the culture solution on the liquid surface and in the region close to the liquid surface from the center of the culture tank toward the inside wall part, the flow of the culture solution from the center of the culture tank toward the inside wall part is generated on the culture solution on the liquid surface and in the region close to the liquid surface. If the flow which is formed on the liquid surface of the culture solution and in the region close to the liquid surface reaches the inside wall of the culture tank, the flow of the culture solution is generated in the direction toward the bottom surface on the outer peripheral portion of the culture tank along the inside wall of the culture tank. Sequentially, the flow of the culture solution is generated in the direction from the outer peripheral portion of the culture tank toward the center portion along the bottom surface of the culture tank, and thereafter, the flow of the culture solution is generated in the direction from the bottom surface at the center portion of the culture tank toward the flow generator which is disposed on the upper portion.

The flow generator generates the flow of the culture solution from the center portion of the culture tank toward the inside wall part on the liquid surface of the culture solution accommodated in the culture tank and in region close to the liquid surface. However, compared to a case in which a deep portion of a culture solution is stirred, since a reaction force generated depending on viscosity of the culture solution, that is, resistance which generates a load with respect to the flow generator, is small, the load applied to the flow generator decreases. Therefore, compared to the related art, it is possible to decrease the consumption power of the flow generator. That is, it is possible to prevent the decay due to the sedimentation or accumulation of the algae and to culture the same amount or more of the algae as the amount of the related art with an amount of power less than that of the culturing apparatus in the related art.

Brief Description of Drawings

FIG. 1 is a plan view showing a schematic configuration of an algae culturing system according an embodiment of the present invention.
FIG. 2 is a sectional view taken along line A-A of the algae culturing system shown in FIG. 1.
FIG. 3 is a sectional view taken along line B-B of the algae culturing system shown in FIG. 1.
FIG. 4 is a sectional view showing another example of an algae recovery line.
FIG. 5 is a sectional view showing a modification example of the algae culturing apparatus of the present invention.

FIG. 6 is a plan view showing a schematic configuration of an algae culturing apparatus disclosed in patent document 1.
FIG. 7 is a sectional view showing a schematic configuration of a stirring device shown in FIG. 6.

Description of Embodiments

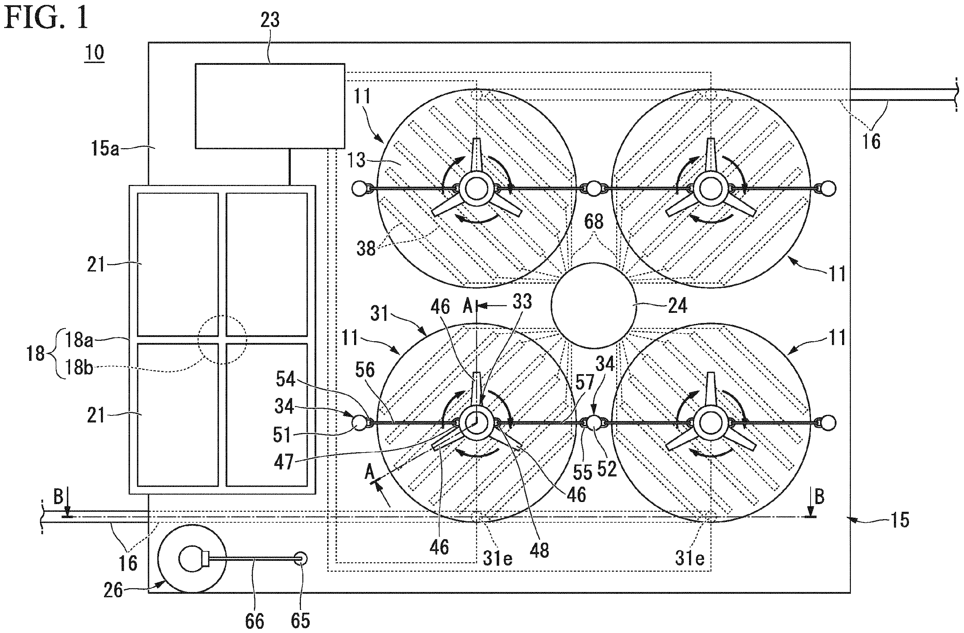

FIG. 1 is a plan view showing a schematic configuration of an algae culturing system according to an embodiment of the present invention.

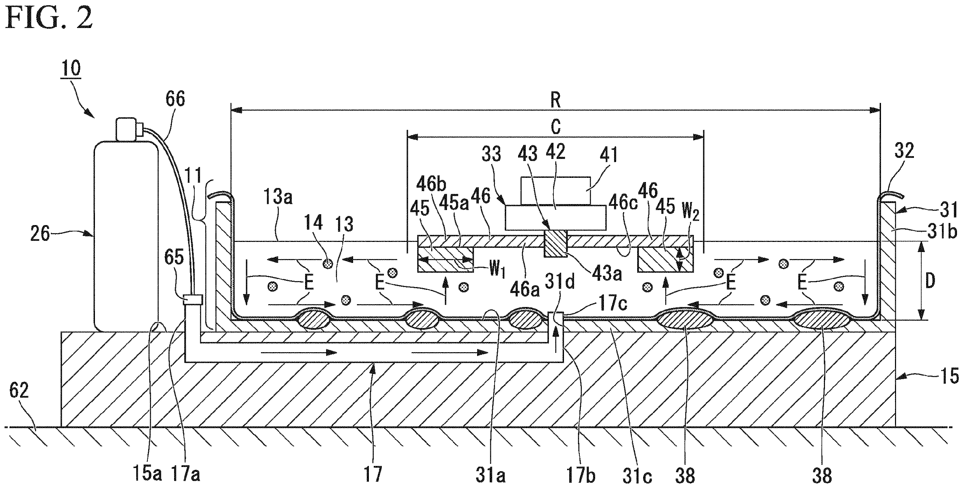

In FIG. 1, the sheet 32 which is described below and is shown in FIG. 2 is not shown. In addition, arrows drawn around flow generators 33 shown in FIG. 1 indicate the rotations of the flow generators 33 with respect to a culture tank 31.

FIG. 2 is a sectional view taken along line A-A of the algae culturing system shown in FIG. 1. In FIG. 2, for easy description, the first end portion 17a, the carbon dioxide supply source 26, the connection member 65, and the carbon dioxide supply line 66 which do not pass through line A-A shown in FIG. 1 are shown.

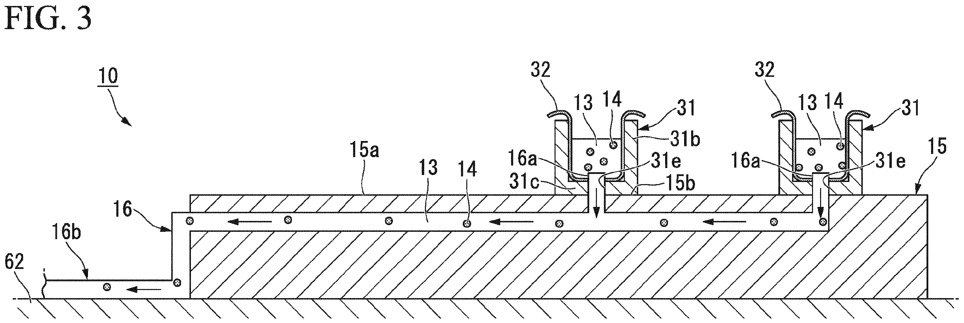

FIG. 3 is a sectional view taken along line B-B of the algae culturing system shown in FIG. 1. In FIGS. 2 and 3, the same reference numerals are assigned to the same components as those of FIG. 1.

Referring to FIGS. 1 to 3, the algae culturing system 10 of the present embodiment includes algae culturing apparatuses 11 (four in the case of FIG. 1), the pedestal portion 15, the algae recovery line 16, the carbon dioxide supply pipe 17, the panel support member 18, the solar cell panel 21, the power storage portion 23, the solar light collection portion 24, and the carbon dioxide supply source 26.

Each of the algae culturing apparatuses 11 includes the culture tank 31 in which algae 14 which are photosynthetic microalgae and the culture solution 13 for culturing the algae 14 are accommodated, the flow generator 33 which generates a predetermined flow in the culture solution 13, the flow generator position regulation member 34 which maintains the flow generator 33 at a predetermined position with respect to the culture tank 31, and the light irradiation member 38 which sends light irradiation to the algae 14 existing in the culture solution 13.

The culture tank 31 includes the inside wall part 31b having a cylindrical shape, and the bottom plate portion 31c which is a circular plate member which covers the lower end of the inside wall part 31b. The sheet 32 is disposed in the culture tank 31 so as to cover the inner surface of the culture tank 31.

The through portion 31d is provided in the center of the bottom plate portion 31c of the culture tank 31, and the second end portion 17b of the carbon dioxide supply pipe 17, which supplies carbon dioxide to the culture solution 13 accommodated in the tank, passes through the through portion 31d. The end portion 17b of the carbon dioxide supply pipe 17 penetrates the sheet 32, and protrudes toward the bottom of the culture tank 31.

In addition, an algae recovery port 31e is provided on the bottom plate portion 31c close to the inside wall part 31b of the culture tank 31, and the end portion of the algae recovery line 16 which recovers the culture solution 13 accommodated in the tank and the cultured algae 14 passes through the algae recovery port 31e. The end portion of the algae recovery line 16 also penetrates the sheet 32, and is exposed to the bottom of the culture tank 31.

In addition, the light irradiation members 38 are disposed on the upper surface side of the bottom plate portion 31c of the culture tank 31, and concave portions for regulating the positions of the light irradiation members 38 are provided.

For example, the inner diameter R (in other words, an inner diameter of the inside wall part 31b) of the culture tank 31 may be appropriately selected within the range of 3 m to 6 m.

A depth D of the culture solution 13 accommodated in the culture tank 31 is smaller than the value of the inner diameter R of the culture tank 31. For example, the depth D of the culture solution 13 accommodated in the culture tank 31 may be appropriately selected within the range from 0.4 m to 1 m. Preferably, the depth D is from 0.4 m to 0.5 m.

Specifically, in a case where the inner diameter R of the culture tank 31 is 5 m, for example, the depth D of the culture solution 13 accommodated in the culture tank 31 may be 0.4 m.

The shape of the inside wall part 31b of the culture tank 31 is not limited to a cylindrical shape, and may be a polygonal shape. In addition, even when the installation site of the system including the algae culturing apparatuses is a tank having a non-uniform shape (a general shape), the subject matter of the present invention is not affected.

That is, in the algae culturing apparatus 11 of the present embodiment, the algae 14 are cultured using the culture solution 13 having a low depth. For example, as the cultured algae 14, microalgae (for example, *Botryococcus, Pseudochoricystis, Chlorella, Spirulina, Scenedesmus, Nannochloropsis, Fistreefeller,* or the like) useable for an algae bio-fuel may be used. In this case, for example, as the culture solution 13, the f/2 medium which is an artificial synthetic medium may be used.

In addition, instead of the culture tank 31, a culture tank may be used, which is constituted of the inside wall part 31b constituting the culture tank 31, and a culturing apparatus placement surface 15b (a surface which functions as the bottom plate portion of the culture tank (specifically, for example, a surface constituted of concrete)) of the pedestal portion 15 in which the inside wall part 31b is disposed.

If the culture tank having the above-described configuration is used, the bottom plate portion 31c is not required. Accordingly, the weight of the algae culturing apparatus 11 can be decreased, and it is possible to easily transport the algae culturing apparatus 11.

The sheet 32 covers the culture tank 31 so as to cover the inner surface of the culture tank 31, accommodates the culture solution including the algae 14 in the culture tank 31, and comes into close contact with the inside wall part 31b and the bottom plate portion 31c. Accordingly, the inside shape of the culture tank 31 is transferred to the sheet 32. In this way, since the culture solution 13 including the algae 14 is accommodated in the culture tank 31 through the sheet 32, water cut-off performance of the culture tank 31 increases, and it is possible to prevent the inner surface of the culture tank 31 from being stained by the algae 14 and the culture solution 13.

As shown in FIG. 2, in a case where the light irradiation members 38 are disposed below the sheet 32, as the sheet 32, a sheet (for example, transparent sheet) having light transmittance may be used.

In this way, since the sheet having light transmittance is used as the sheet 32, light (for example, solar light) emitted from the light irradiation members 38 can be emitted to the algae 14 existing in the culture solution 13 accommodated in the sheet 32.

In addition, in FIG. 2, for example, a case where the light irradiation members 38 are disposed between the sheet 32 and the bottom plate portion 31c is described. However, the disposition positions of the light irradiation members 38 are not limited to this configuration. The light irradiation members 38 may be disposed on the sheet 32 so as to be immersed into the culture solution 13. In this case, since the algae 14 existing in the culture solution 13 can be irradiated with light without the light passing through the sheet 32, the sheet 32 does not need to have light transmittance. Accordingly, as the sheet 32, various kinds of sheets may be used.

In addition, in FIGS. 2 and 3, for example, the case where the algae 14 and the culture solution 13 are accommodated in the culture tank 31 through the sheet 32 is shown. However, the algae 14 and the culture solution 13 may be directly accommodated in the culture tank 31 without using the sheet 32.

The flow generator 33 is disposed at the center portion C of the culture tank 31 in a state (refer to FIG. 2) where the flow generator 33 floats on the culture solution 13 accommodated in the culture tank 31 through the sheet 32. Since the flow generator 33 moves the culture solution 13 in the region close to the liquid surface 13a from the center portion C of the culture tank 31 toward the inside wall part 31b, the flow from the center portion C of the culture tank 31 toward the inside wall part 31b is omnidirectionally generated in the culture solution 13 in the region close to the liquid surface 13a.

Only one flow generator 33 is disposed with respect to one culture tank 31. In this way, since one flow generator 33 is disposed with respect to one culture tank 31, unlike a case where the flow generators 33 arc disposed with respect to one culture tank 31, it is possible to culture a predetermined amount of algae 14 with less power.

The flow generator 33 includes the motor 41, the speed reducer 42, the rotary shaft 43, blade members 45 (three in the case of the algae culturing apparatus 11 shown in FIG. 1), support members 46 (three in the case of the algae culturing apparatus 11 shown in FIG. 1), each of which supports each of the blade members 45, and rope fixing portions 47 and 48.

The rope fixing portions 47 and 48 are provided on an outer wall of the speed reducer 42. The rope fixing portions 47 and 48 are disposed so as to face each other through the speed reducer 42. The rope fixing portion 47 is a member for connecting one end of a first rope 56 (one rope for regulating the position of the flow generator 33) described below. The rope fixing portion 48 is a member for connecting one end of the second rope 57 (the other rope for regulating the position of the flow generator 33) described below.

The motor 41 is connected to first end portion of the rotary shaft 43 through the speed reducer 42. When the flow generator 33 floats on the culture solution 13, the rotary shaft 43 is provided such that the axial direction of the rotary shaft 43 is orthogonal to the liquid surface 13a in which the flow of the culture solution 13 is not formed.

A base end portion 46a of the support member 46 is connected to the second end portion 43a of the rotary shaft 43, and the blade member 45 is attached to the lower surface 46c of the tip portion 46b. The support member 46 has a resin hollow structure, specific weight of the support member 46 is significantly lighter than that of the culture solution 13, and the support member 46 functions as a float (a member which generates buoyancy) of the flow generator 33. In addition, the structure of the support member 46 is not limited to the resin hollow structure, and may adopt any structure as long as it can generate sufficient buoyancy in the flow generator 33.

In addition, the support members 46 are disposed such that all angles between adjacent support members 46 are the same as each other. In the case of the structure shown in FIG. 1, three support members 46 are disposed such that adjacent support members 46 are separated from each other by 120°. Accordingly, when the flow generator 33 floats on the culture solution 13, the motor 41 and the speed reducer 42 are maintained above the liquid surface 13a while the blade member 45 attached to the lower surface 46c of the support member 46 is disposed at a region which is relatively shallow downward from the initial liquid surface 13a of the culture solution 13. Therefore, the flow generator 33 can float on the culture solution 13 in a stable state.

When the motor 41 and the speed reducer 42 are disposed at the approximately center of the culture tank 31 and the flow generator 33 floats on the culture solution 13, the blade members 45 are disposed at the positions separated from the center of the culture tank 31 so as to be close to the inside wall part 31b. In addition, the blade member 45 is attached to the support member 46 such that the length direction of the blade member 45 matches with that of the support member 46 and the width direction of the blade member 45 is orthogonal to the liquid surface 13a of the culture solution 13.

The culture solution 13 is a liquid mixture of photosynthetic microalgae and a culture solution, and has predetermined viscosity as a fluid. Here, the viscosity means characteristics in which a force (reaction force) pushes back movements of a material which is a factor of the velocity gradient in proportion to the velocity gradient with respect to the material when the velocity gradient is generated in the fluid. In the present embodiment, the support members 46 and the blade members 45 (mainly, blade members 45) which rotate in the culture solution 13 are factors of the velocity gradient.

In the algae culturing apparatus (in Japanese Unexamined Patent Application, First Publication No. 2011-254724) of the related art, since the propeller, which is the key factor for the velocity gradient, is positioned away from the liquid surface of the culture solution, the viscosity of the culture solution which generates the reaction force with respect to the rotation of the propeller is applied to all portions including the upper portion and the lower portion of the propeller.

Meanwhile, in the present embodiment, since the blade members 45 is disposed at the region which is relatively shallow downward from the liquid surface 13a of the culture solution 13, the viscosity of the culture solution which generates the reaction force with respect to the rotations of the support member 46 and the blade member 45 is obviously smaller than the viscosity of the algae culturing apparatus in the related art. This is because the upper portion of the blade member 45 is close to the liquid surface 13a and the velocity gradient generated in the culture solution between the blade member 45 and the liquid surface 13a decreases. The culture solution 13 comes into contact with the air through the liquid surface 13a. However, compared to the viscosity of the culture solution 13, the viscosity of the air can be ignored since the viscosity of the air is significantly small.

That is, in the present embodiment, since the reaction force (the force which pushes back the rotating blade member 45) generated depending on the viscosity of the culture solution is smaller than that of the algae culturing apparatus in the related art, a load, which is applied to the motor 41 which rotates the blade members 45, decreases. Accordingly, compared to the related art, the amount of power consumed by the motor 41, that is, the flow generator 33, can be dccrcascd.

If the viscosity of the culture solution 13 is considered, as described above, when flows are generated in the culture solution close to the liquid surface 13a rather than when the culture solution 13 is stirred in the region close to the bottom of the culture tank 31, the amount of power consumed decreases. Next, if the motor 41 is driven and the support members 46 and the blade members 45 rotate in the state where the motor 41 and the speed reducer 42 are disposed at the approximately center of the culture tank 31 and the flow generator 33 floats on the culture solution 13, the culture solution which is close to the liquid surface 13a and exists on the front side in the rotation direction of the blade member 45 is pressed by the blade member 45, and performs a circular movement about the rotary shaft 43 of the motor 41. However, since the culture solution is not restricted except for own viscosity in the radial direction in the liquid, a flow is generated in the culture solution by centrifugal force which is applied from the center toward the outside in the radial direction according to the circular movement. At this time, since the motor 41 and the speed reducer 42 are disposed at the approximately center of the culture tank 31, as a result, the flow from the center portion C of the culture tank 31 toward the inside wall part 31b is generated in the culture solution 13 in the region close to the liquid surface 13a.

If the flow generated in the culture solution 13 close to the liquid surface 13a reaches the inside wall part 31b, the direction of the flow is changed, and the flow is directed downward along the inside wall part 31b. If the flow reaches the bottom surface 31a of the culture tank 31, the direction of the flow is changed, and the flow is directed to the center of the tank from the inside wall part 31b. The culture solution reaching the center of the tank is omnidirectionally diffused by the driving of the flow generator 33, and rises toward the liquid surface in the center of the tank along with the culture solution which reaches the center of the tank through the same process. Accordingly, since the flow of the culture solution circulating in the vertical half section in the radial direction of the culture tank 31 is generated, it is possible to effectively culture the algae while preventing decay due to sedimentation or accumulation of the algae in the culture tank 31.

For example, as the blade member 45, a rectangular blade may be used. In a case where a rectangular blade is used as the blade member 45, the inner diameter R of the culture tank 31 is 5 m, and the depth D of the culture solution 13 is 0.4 m, for example, the length $W_1$ (the length of the blade member 45 in the radial direction of the culture tank 31) of the blade member 45 may be set to 30 cm. In addition, for example, the width (the length of the blade member 45 parallel in the depth direction of the culture tank 31) $W_2$ of the blade member 45 may be set to 25 cm.

For example, as a material which configures the blade member 45, wood, plastic, stainless steel may be used.

Preferably, the length of the support member 46 is within a range of 30% to 40% of 1/2 (in other words, a radius r expressed in the following equation (5)) of the inner diameter R. Specifically, in a case where the value of 1/2 of the inner diameter R is 2.5 m, the length of the support member 46 may be appropriately set within a range of 0.75 m to 1.0 m.

In addition, in a case where the motor 41 having a large output is used, the number of the support members 46 may increase according to the intensity of the output. In this case, for example, the angle between adjacent support members 46 decreases so as to be 90° or 60° according to the number of the support members 46 in order to maintain the equal interval disposition of the support members 46.

In a case where the motor 41 having a large output is used, there is a concern that weight of the motor 41 and the speed reducer 42 may increase. However, since the buoyancy of the flow generator 33 with respect to the increase in weight of the motor 41 and the speed reducer 42 is secured by increasing the number of the support members 46 serving as the float, the flow generator 33 can float on the culture solution 13 without impairing the stable state.

As described above, since the support members 46 are disposed such that the angles between the adjacent support members 46 are the same as each other, it is possible to uniformly generate the radial flows which are omnidirectionally diffused from the center portion C of the culture tank 31 toward the inside wall part 31b of the culture tank 31 by the blade members 45 attached to the support members 46.

Here, the flow rate v of the culture solution 13 on the outer peripheral edge in the culture tank 31 which is required for preventing precipitation or extinction of the algae 14 is described.

Input energy efficiency φ per volume (hereinafter, referred to as "culture volume") of the culture solution 13 which cultures the algae 14 is expressed by the following equation (2).

In addition, the input energy efficiency φ is the parameter which is obtained by dividing the culture volume by the output (feed output) of the flow generator required for culturing the algae 14, and is a parameter which indicates how much the culture solution 13 is stirred by the feed output.

$$\phi = V/Watt \qquad \cdots (2)$$

In the equation (2), V is the culture volume (hereinafter, referred to as "culture volume V"), and Watt is the feed output of the flow generator 33, that is, the output (hereinafter, referred to as "feed output Watt") which is used so as to stir the culture solution 13.

At this time, $m^3$ (cubic meters) is used as the unit of the culture volume V, and W (watt) is used as the unit of the feed output Watt.

In the input energy efficiency φ expressed by the equation (2), the energy efficiency increases as the value of input energy efficiency increases.

For example, preferably, the input energy efficiency φ has 0.20 $m^3$/W to 0.32 $m^3$/W as the target value. More preferably, the input energy efficiency φ has a value more than the numerical range.

In addition, from the viewpoint of preventing the precipitation or the extinction of the algae 14, in order to increase the culture efficiency of the algae 14, the flow rate of the stirred culture solution 13 is important. Here, if the equation (2) is rearranged, the following the equation (3) is obtained.

$$V = \phi \cdot \text{Watt} \qquad \cdots (3)$$

Here, the culture volume V is expressed by the following equation (4) using the depth D of the culture solution 13 accommodated in the sheet 32 and the radius r (value of 1/2 of the inner diameter R) of the culture tank 31. For example, the depth D of the culture solution 13 (the unit is m (meter)) can be appropriately selected within the range of 0.4 m to 1 m. Preferably, the depth D is 0.4 m to 0.5 m.

$$V = D \cdot \pi r^2 \qquad \cdots (4)$$

The following equation (5) is obtained from the equation (3) and the equation (4).

$$\phi \cdot \text{Watt} = D \cdot \pi r^2 \qquad \cdots (5)$$

If he equation (5) is rearranged with respect to the radius r of the culture tank 31, the following the equation (6) is obtained. ⌐

$$r = \sqrt{\frac{\phi \cdot \text{Watt}}{D \cdot \pi}} \qquad \cdots (6)$$

The flow rate v of the culture solution 13 on the outer peripheral edge in the culture tank 31 can be expressed by the following equation (7). In addition, t expressed in the equation (7) indicates a time (hereinafter, referred to as a "time t") required for the culture solution 13 completing one revolution around the outer peripheral edge in the culture tank 31. The time t is a value which is actually measured, and uses minutes as the unit.

$$v = 2\pi r / t \qquad \cdots (7)$$

If the radius r of the culture tank 31 in the equation (7) is replaced by the radius r of the culture tank 31 expressed by the equation (6), the following equation (8) is obtained.

$$v = 2\pi \frac{\sqrt{\frac{\phi \cdot \text{Watt}}{D \cdot \pi}}}{t} \qquad \cdots (8)$$

By setting the target value of the input energy efficiency φ, setting the depth D of the culture solution 13 within the above-described range, and obtaining the time t by measuring the time t, it is possible to obtain the flow rate v of the culture solution 13 on the outer peripheral edge in the culture tank 31 by the equation (8).

In a case where an open-pond type algae culturing apparatus of the related art is used, if the flow rate of the culture solution is 12 m/min or more, it is possible to prevent the precipitation or extinction of the algae.

Accordingly, preferably, the flow rate v of the culture solution 13 on the outer peripheral edge in the culture tank 31 is 12 m/min or more.

As described above, in the algae culturing apparatus 11 of the present embodiment, the inside wall part has a cylindrical shape, and the algae culturing apparatus 11 includes the culture tank 31 in which the algae 14 and the culture solution 13 culturing the algae 14 are accommodated through the sheet 32, and the flow generator 33 which is disposed at the center portion C of the culture tank 31 in a state where the flow generator 33 floats on the culture solution 13. Since the flow generator 33 moves the liquid surface in the culture tank 31 and the culture solution 13 in the region close to the liquid surface from the center portion C of the culture tank 31 toward the inside wall part 31b of the culture tank 31, the flow of the culture solution 13 from the center portion C of the culture tank 31 toward the inside wall part 31b of the culture tank 31 is generated on the liquid surface of the culture solution 13 and in the region closet to the liquid surface. If the flow which is formed on the liquid surface 13a of the culture solution 13 and in the region close to the liquid surface 13a reaches the inside wall part 31b of the culture tank 31, the flow of the culture solution 13 is generated in the direction toward the bottom surface 31a on the outer peripheral portion of the culture tank 31 along the inside wall part 31b of the culture solution 13. Sequentially, the flow of the culture solution 13 is generated in the direction from the outer peripheral portion of the culture tank 31 toward the center portion C along the bottom surface 31a of the culture tank 31, and thereafter, the flow of the culture solution 13 is generated in the direction from the bottom surface 31a at the center portion C of the culture tank 31 toward the flow generator 33 which is disposed on the upper portion.

The flow generator 33 generates the flow of the culture solution 13 from the center portion C of the culture tank 31 toward the inside wall part 31b on the liquid surface of the culture tank 31 and the vicinity of the liquid surface. However, since the blade member 45 is disposed at the region which is relatively shallow downward from the liquid surface 13a of the culture solution 13, compared to the algae culturing apparatus of the related art in which a deep portion of a culture solution is stirred, the viscosity of the culture solution which generates the reaction force with respect to the rotations of the support member 46 and the blade member 45 is small. Accordingly, the load applied to the motor 41 which rotates the blade member 45 decreases. Therefore, compared to the related art, it is possible to decrease the consumption power of the motor 41, that is, the flow generator 33.

That is, it is possible to prevent the decay due to the sedimentation or accumulation of the algae 14 and to culture a predetermined amount of algae with an amount of power less than that of the culturing apparatus in the related art.

According to the effects, as the motor 41 which is used when the blade member 45 rotates, a motor in which the maximum output is 25W (that is, a motor in which the consumption power is small) may be used.

The flow generator position regulation member 34 includes a first post 51, a second post 52, a first rope fixing portion 54, a second rope fixing portion 55, a first rope 56, and a second rope 57.

The first and second posts 51 and 52 are provided on the upper surface side of the pedestal portion 15 such that the posts face each other in a state where the culture tank 31 is interposed therebetween. The first and second posts 51 and 52 protrude upward from the upper surface 15a of the pedestal portion 15.

The first rope fixing portion 54 is provided on a portion of the outer wall of the first post 51 facing the second post 52. The second rope fixing portion 55 is provided on a portion of the outer wall of the second post 52 facing the first post 51.

One end of the first rope 56 is connected to the rope fixing portion 47, and the other end thereof is connected to the first rope fixing portion 54. One end of the second rope 57 is connected to the rope fixing portion 48, and the other end thereof is connected to the second rope fixing portion 55.

In this way, since the flow generator position regulation member 34 for regulating the position of the flow generator 33 is provided, even in a case where the flow generator 33 is operated in a state where the flow generator 33 floats on the culture solution 13 accommodated in the culture tank 31, it is possible to always dispose the flow generator 33 at the center portion C of the culture tank 31. Accordingly, it is possible to decrease deviation according to a place in a circulation flow E which is formed in the culture solution 13 accommodated in the culture tank 31.

When the culture of the algae 14 proceeds and the solar light does not reach from the liquid surface 13a side to the sheet 32 disposed on the bottom surface 31a of the culture tank 31, the light irradiation member 38 is a member for irradiating the algae 14 during culture with the solar light required from photosynthesis.

For example, as the light irradiation member 38, a member may be used which includes an optical fiber (not shown) which emits the solar light, and a light transmitting tube having an optical fiber accommodation portion (not shown) capable of accommodating the optical fiber (not shown) in the inner portion of the light transmitting tube.

In the case of this configuration, the optical fiber sends solar light irradiation to the algae 14 accommodated in the sheet 32 through the optical fiber accommodation portion (not shown). Accordingly, the optical fiber accommodation portion (not shown) which is configured of a light transmitting material may be used.

For example, as the material of the optical fiber accommodation portion (not shown), a light transmitting resin (for example, acrylic resin, polycarbonate resin, or the like), glass, or the like may be used.

For example, the light irradiation members 38 having the above-described configuration may be disposed according to a layout shown in FIG. 1.

The layout of the light irradiation members 38 shown in FIG. 1 is an example, and the present invention is not limited to this.

In addition, the light irradiation member 38 may include any member as long as it can send light irradiation to the algae 14 in the culture with the light required for photosynthesis, and is not limited to the member which includes the optical fiber as the component. In addition, in the present embodiment, the solar light serving as the external light source is emitted to the algae 14 through the optical fiber. However, the present invention is not limited to the solar light, and the photosynthesis of the algae may be promoted by light of an artificial light source such as a fluorescent lamp or LED. In a case where the artificial light source such as the fluorescent lamp or LED is used as the light irradiation member 38, the artificial light source may be disposed on the upper surface side of the bottom plate portion 31c of the culture tank 31 and the light may be directly emitted to the algae 14 without the light passing through the optical fiber.

In addition, in FIG. 1, as an example of the algae culturing system 10, the case where the algae culturing system 10 includes four algae culturing apparatuses 11 is described. However, the number of the algae culturing apparatuses 11 configuring the algae culturing system 10 is not limited to this description as long as the number is multiple. In addition, for example, the number of the algae culturing apparatuses 11 configuring the algae culturing system 10 may be appropriately selected according to the inner diameter R of the culture tank 31.

The pedestal portion 15 is a member on which the algae culturing apparatuses 11, the algae recovery line 16, the panel support member 18, the solar cell panel 21, the power storage portion 23, the solar light collection portion 24, and the carbon dioxide supply source 26 are disposed. The pedestal portion 15 is disposed on the base surface 62.

The upper surface 15a of the pedestal portion 15 is a flat surface. The upper surface 15a of the pedestal portion 15 includes a plurality of culturing apparatus placement surfaces 15b on which the algae culturing apparatuses 11 are placed. Each of the culturing apparatus placement surfaces 15b is a circular surface. In the case of FIG. 1, two culturing apparatus placement surfaces 15b are disposed so as to be adjacent to each other with respect to a vertical direction and a lateral direction.

For example, as the material of the pedestal portion 15 having the above-described configuration, concrete may be used. In addition, since a portion of the algae recovery line 16 is provided inside the pedestal portion 15, for example, the thickness (height) of the pedestal portion 15 may be 0.2 m to 0.5 m.

The algae recovery line 16 is a line through which the algae 14 cultured in the sheets 32 of the culture tanks 31 are discharged through the bottom plate portion 31c of the culture tank 31 along with the culture solution 13 so as to recover the cultured algae 14.

A portion of the algae recovery line 16 is provided inside the pedestal portion 15, and the remaining portion thereof is disposed outside the pedestal portion 15 (including the portion disposed on the base surface 62). In the algae recovery line 16, the portion (including first end portion 16a of the algae recovery line 16) provided inside the pedestal portion 15 is disposed at a higher position than that of the portion (including the second end portion 16b of the algae recovery line 16) which is disposed on the base surface 62.

The first end portion 16a (the end portion which is disposed inside the pedestal portion 15) of the algae recovery line 16 is inserted into the algae recovery port 31e. The first end portion 16a of the algae recovery line 16 protrudes upward from the bottom plate portion 31c, and penetrates the center portion of the sheet 32.

Accordingly, the algae recovery line 16 is configured so as to recover the culture solution 13 and the algae 14 which are accommodated in the sheets 32.

The algae recovery line 16 having the above-described configuration is configured so as to recover the algae 14 utilizing height difference between the first end portion 16a of the algae recovery line 16 and the second end portion 16b of the algae recovery line 16 which is disposed at the lower position than the first end portion 16a. Accordingly, since power for recovering the cultured algae 14 is not required, it is possible to save power.

The first end portion 17a of the carbon dioxide supply pipe 17 protrudes from the upper surface 15a of the pedestal portion 15, and other portions thereof are provided inside the pedestal portion 15. The connection member 65 which is a member connected to the carbon dioxide supply source 26 is mounted on the first end portion 17a of the carbon dioxide supply pipe 17 through a carbon dioxide supply line 66.

The second end portion 17b (the end portion provided inside the pedestal portion 15) of the carbon dioxide supply pipe 17 is inserted into the through portion 31d. A carbon dioxide supply port 17c which is disposed on the tip of the second end portion 17b of the carbon dioxide supply pipe 17 protrudes upward from the bottom plate portion 31c, and penetrates a portion of the sheet 32.

Accordingly, the carbon dioxide supply pipe 17 supplies the carbon dioxide filled in the carbon dioxide supply source 26 to the algae 14 existing in the culture solution 13 through the carbon dioxide supply port 17c.

The panel support member 18 includes a support column 18a and a panel support body 18b. The first end portion of the support column 18a is fixed to the pedestal portion 15, and protrudes upward from the upper surface 15a of the pedestal portion 15. The second end portion of the support column 18a is connected to the panel support body 18b. Accordingly, the support column 18a supports the panel support body 18b.

The panel support body 18b is a support body to which solar cell panels 21 (in the case of FIG. 1, four solar cell panels 21) are attached. The panel support body 18b includes a panel fixing surface (not shown).

In addition, a drive mechanism (not shown) for adjusting the direction of the panel support body 18b configuring the panel support member 18 or an inclination angle with respect to the upper surface 15a of the pedestal portion 15 may be provided. In this way, since the drive mechanism (not shown) is provided, the solar cell panel 21 can be effectively irradiated with the solar light, and it is possible to effectively obtain power.

The solar cell panels 21 are fixed to the panel fixing surfaces (not shown) of the panel support body 18b. The solar cell panels 21 (four solar cell panels in the case of FIG. 1) are electrically connected to the power storage portion 23.

In a case where the algae 14 are cultured using the algae culturing system 10 (the algae culturing system 10 having the configuration shown in FIG. 1) having four flow generators 33 including the motors 41 in which the maximum output is 25W, for example, as the solar cell panels 21, four panels of 220W may be used. Accordingly, during the daytime, it is possible to obtain an amount of power equal to or more than the amount of power which is used by the motors 41.

The power storage portion 23 is disposed on the pedestal portion 15. The power storage portion 23 stores the power which is converted by the plurality solar cell panels 21. The power storage portion 23 is electrically connected to the flow generators 33 in a state where the power storage portion 23 can supply power to the flow generators 33.

In this way, since the power storage portion 23 is provided, which stores the power which is not used during the daytime in the power converted by the solar cell panels 21, it is possible to operate the algae culturing system 10 during the night using the power stored in the power storage portion 23 without preparing separate power.

For example, as the power storage portion 23, a combination of a plurality of batteries used in a truck which is a vehicle may be used.

The solar light collection portion 24 is provided on the pedestal portion 15 which is surrounded by the algae culturing apparatuses 11. The solar light collection portion 24 is connected to a plurality of solar light transport lines 68 which are connected to the light irradiation members 38 (specifically, in the case of the present embodiment, the structure configured of the optical fibers and the optical fiber accommodation portions accommodating the optical fibers).

The solar light collection portion 24 supplies the collected solar light to the light irradiation members 38 configuring the algae culturing apparatuses 11 through the solar light transport lines 68.

The carbon dioxide supply source 26 is fixed to the pedestal portion 15 by a carbon dioxide supply source fixing member (not shown). The carbon dioxide supply source 26 supplies carbon dioxide required for photosynthesis to the algae 14 through the carbon dioxide supply line 66 and the carbon dioxide supply pipe 17 (including the carbon dioxide supply port 17c).

For example, as the carbon dioxide supply source 26, a high pressure gas cylinder which is filled with carbon dioxide may be used.

The algae culturing system of the present embodiment includes the algae culturing apparatuses 11, the pedestal portion 15 which includes the flat culturing apparatus placement surfaces 15b on which the algae culturing apparatuses 11 are placed, the power storage portion 23 which is disposed on the pedestal portion 15 and is electrically connected to the algae culturing apparatuses 11, and the solar cell panel 21 which is electrically connected to the power storage portion 23. According to the algae culturing system of the present embodiment, since the power (the power for driving the flow generators 33) required for the algae culturing apparatuses 11 when the algae 14 is cultured with an amount of power less than that of the related art can be covered by the power generated by the solar cell panel 21, unlike the algae culturing system of the related art which is operated by the power purchased from an electric power company, it is possible to culture a predetermined amount of algae at a low cost.

In addition, since the power required for the algae culturing system 10 can be supplied by the algae culturing system 10 single body, even in a place at which power is not easily supplied, the algae culturing system 10 is installed and the algae 14 can be cultured as long as the place has good sunlight conditions.

The algae culturing system 10 including the algae culturing apparatuses 11 can obtain effects similar to those of the above-described algae culturing apparatus 11.

Hereinbefore, the preferred embodiment of the present invention is described. The present invention is not limited to the specific embodiment, and various deformation and modifications may be applied to the present invention within the scope of the gist of the present invention described in claims.

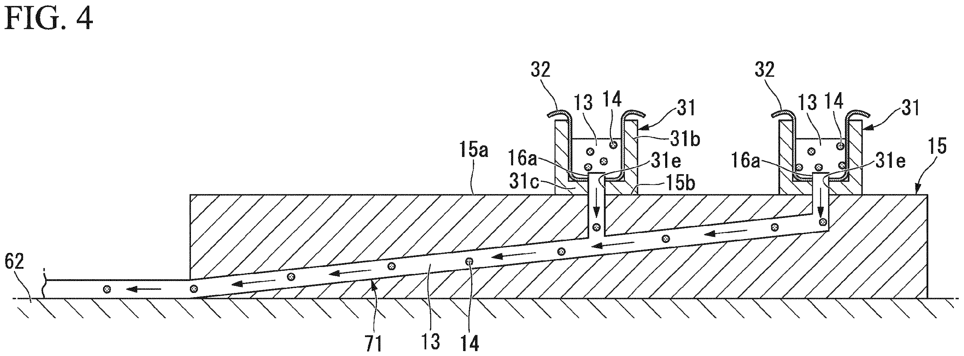

For example, in the algae culturing system 10 of the present invention, the case where four culture tanks 31 are provided is described. However, the number of the culture tanks 31 is not limited to this. In addition, an algae recovery line 71 which is described below and shown in FIG. 4 may be used. In addition, the solar cell panels 21 may be installed above the culture tank 31.

FIG. 4 is a sectional view showing another example of the algae recovery line. In FIG. 4, the same reference numerals are assigned to the same portions of the structure shown in FIG. 3.

The structure shown in FIG. 4 is the same as the structure shown in FIG. 3 except that the algae recovery line 71 is provided instead of the algae recovery line 16 which is the component of the structure shown in FIG. 3.

In FIG. 3, the case is described, in which the algae recovery line 16 is provided inside the pedestal portion 15 such that the center axis of the portion of the algae recovery line 16 extending toward the outer peripheral portion of the pedestal portion 15 and the upper surface 15a of the pedestal portion 15 are parallel with each other. However, for example, as shown in FIG. 4, the algae recovery line 71 may be provided inside the pedestal portion 15 such that the center axis of the portion of the algae recovery line 71 extending toward the outer peripheral portion of the pedestal portion 15 intersects the upper surface 15a of the pedestal portion 15.

Compared to the case where the algae recovery line 16 shown in FIG. 3 is provided, in the algae recovery line 71 having the above-described configuration, it is possible to easily recover the algae 14 cultured in the culture tanks 31 and the culture solution 13.

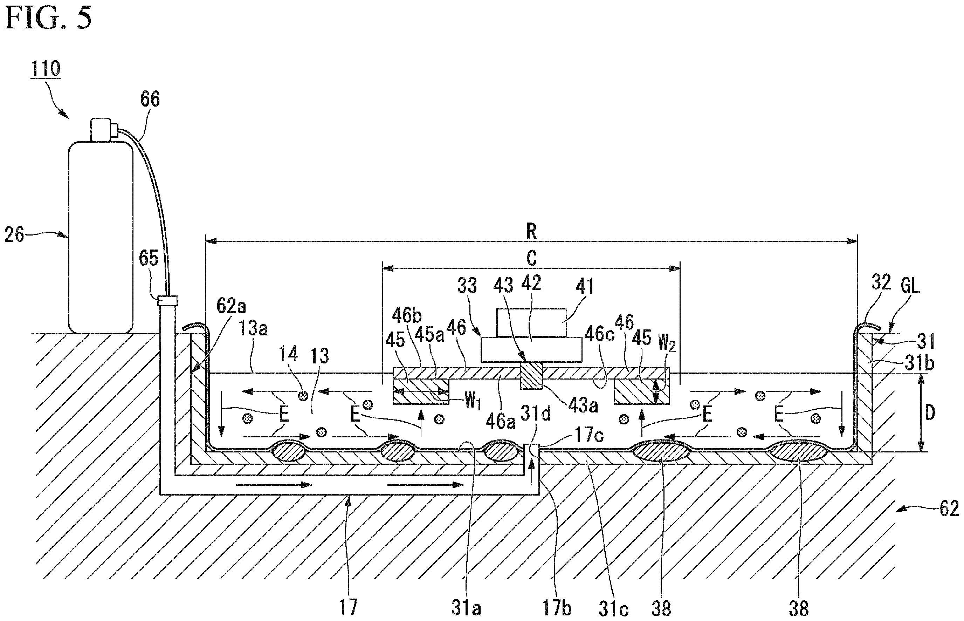

FIG. 5 shows a modification example of the algae culturing apparatus of the present invention. In an algae culturing apparatus 110, the culture tank 31 is provided in a concave portion 62a in which the base surface 62 is dug so as to be deeper than the ground level GL. In this way, since the culture tank 31 is provided at a position lower than the ground level GL, a material such as the pedestal portion can be omitted. In addition, since the inside wall part 31b of the culture tank 31 is supported by a mud wall perpendicular to the concave portion 62a, even though self-sustainable and sufficient strength is not provided to the inside wall part 31b, it is possible to maintain the shape of the culture tank 31. Accordingly, since it is possible to culture algae in a place which is suitable for the culture of the algae but is far away from a consumer, it is possible to structure an algae culturing system with a very small amount of materials.

[Example]

In Example, the algae 14 were cultured using the algae culturing system 10 shown in FIG. 1.

The inner diameter R of the culture tank 31 was set to 5 m. As the sheet, a sheet which was formed of transparent polyvinyl chloride and had a thickness of 3 mm was used.

The f/2 medium having improved components which were the culture solution 13 was supplied to the culture tank 31, and the depth D of the culture solution 13 in the culture tank 31 was set to 0.4 m. In addition, the temperature of the culture solution 13 during the culture was set to a range of 15°C to 35°C.

As the algae 14, 2% to 5% of High Oil Marine Diatom *Fistulifera sp. JPCC DA0580* with respect to the amount of medium was inoculated in the culture solution 13.

The length of each of three support members 46 was set to 1 m. In addition, as the blade member 45, a stainless steel blade member 45 was used, in which the lateral width $W_1$ was 30 cm, the vertical width $W_2$ was 25 cm, and the thickness was 2 mm.

As the motor 41 constituting each flow generator 33, an AC motor in which the maximum output was 25 W was used. In addition, the rotating speed of the support member 46 was set to 6 rpm (six revolutions per one minute).

Thereafter, the culture of the algae 14 started by rotationally driving the flow generator 33 disposed inside each culture tank 31 using power obtained from the solar cell panels 21.

The culture period of the algae 14 was set to 10 days (specifically, 24 hours × 10 = 240 hours). In addition, the power obtained from four solar cell panels 21 was stored in the power storage portion 23, and the algae 14 was cultured during 10 days using this power.

As the culture proceeded, concentration of the algae 14 increased, and light required for photosynthesis did not reach the culture solution 13 positioned in the close to the bottom of the sheet 32.

Accordingly, on the seventh day from the start of the culture, the solar light collected by the solar light collection portion 24 was emitted from the bottom of the culture tank 31.

Therefore, by supplementing shortage of light, productivity of algae 14 and productivity of oil was improved.

However, as the culture of the algae 14 proceeded, pH of the culture solution 13 increased. Accordingly, in a stage at which pH reached 9.0, carbon dioxide was supplied to the culture solution 13.

Therefore, carbon dioxide was dissolved in the culture solution 13 as carbonic acid, and pH of the culture solution 13 was decreased to 8.2.

In addition, the carbon dioxide supplied to the culture solution 13 was also used as a C source (in other words, carbon) required for photosynthesis of the algae 14.

The total power (in other words, total power used to operate four algae culturing apparatuses 11) used in Example was 10 kWh. In addition, the weight of the algae 14 obtained during 10 days was 2 kg.

Accordingly, it was demonstrated that the power required for culturing the algae of 1 kg (a predetermined amount) was 5 kWh.

Industrial Applicability

The present invention can be applied to an algae culturing apparatus and an algae culturing system capable of preventing the decay due to sedimentation or accumulation of the algae and culturing the algae.

Reference Signs List

10: algae culturing system
11: algae culturing apparatus
13: culture solution
13a: liquid surface
14: algae
15: pedestal portion
15a: upper surface
15b: culturing apparatus placement surface
16: algae recovery line
17: carbon dioxide supply pipe
18: panel support member
21: solar cell panel
23: power storage portion
24: solar light collection portion
26: carbon dioxide supply source
31: culture tank
31a: bottom surface
32: sheet
33: flow generator
34: flow generator position regulation member
38: light irradiation member
41: motor
42: speed reducer
43: rotary shaft
45: blade member
45a: upper end surface
46: support member
47, 48: rope fixing portion
51: first post
52: second post
54: first rope fixing portion
55: second rope fixing portion
56: first rope
57: second rope
62: base surface
65: connection member
66: carbon dioxide supply line
68: solar light transport line
71: algae recovery line
110: algae culturing system
C: center portion
D: depth
E: circulation flow
N, $W_1$, $W_2$: width
M: center line
R: inner diameter
GL: ground level

**Claims**

1. An algae culturing apparatus (11), comprising:

    a culture tank (31) configured to accommodate algae and a culture solution for culturing the algae; and
    a flow generator (33) installed on the culture tank (31), and configured to generate a flow of the culture solution from the center of the culture tank (31) toward an inside wall part of the culture tank (31) on a liquid surface of the culture solution and in a region of the culture solution close to the liquid surface by moving the culture solution on the liquid surface and in the region close to the liquid surface in the culture tank (31) from the center of the culture tank (31) toward the inside wall part of the culture tank (31), wherein the flow generator (33) includes:

    a motor (41);
    a rotary shaft (43), one end portion of which is connected to the motor (41);
    a plurality of support members (46) radially disposed about the rotary shaft (43), each of the plurality of support member (46) being connected to other end portion of the rotary shaft (43) on a base end portion thereof; and
    a plurality of blade members (45) which are attached to tip portions of the support members (46), and wherein
    in a state where the rotary shaft (43) is disposed so as to be orthogonal to the liquid surface of the culture solution accommodated in the culture tank (31), the plurality of blade members (45) are disposed so as to be closer to the inside wall part of the culture tank (31) than the rotary shaft (43), and are suspended from the support members (46) to come into contact with the culture solution,
    a specific weight of the support member (46) is lighter than that of the culture solution, and the support member (46) is a floating buoyant material for securing buoyancy of the flow generator (33) with respect to the culture solution, and
    the algae culturing apparatus (11) further comprises a flow generator position regulation member (34) which is configured to regulate a position of the flow generator (33) at the center portion of the culture tank (31) in a state where the flow generator (33) floats on the culture solution.

2. The algae culturing apparatus (11) according to claim 1,
    wherein the plurality of support members (46) are disposed such that all angles between adjacent support members (46) are the same as each other.

3. The algae culturing apparatus (11) according to claim 1 or 2,
    wherein in the culture tank (31), the inside wall part has a cylindrical shape in a plan view, and the flow generator (33) is disposed at the center portion of the culture tank (31).

4. The algae culturing apparatus (11) according to any one of claims 1 to 3,
    wherein the flow generator (33) is configured to generate the flow of the culture solution from the center of the culture tank (31) toward the inside wall part in all directions from the center of the culture tank (31) with respect to the culture solution.

5. The algae culturing apparatus (11) according to any one of claims 1 to 4, further comprising a light irradiation member (38) which sends light irradiation to the algae existing in the culture solution accommodated in the culture tank (31).

6. The algae culturing apparatus (11) according to claim 5,
    wherein the light irradiation member (38) includes an optical fiber which is disposed on a bottom surface of the culture tank (31), guides light from an external light source into the bottom surface of the culture tank (31) through the optical fiber, and sends light irradiation to the algae.

7. The algae culturing apparatus (11) according to claim 5,
    wherein the light irradiation member (38) is disposed on a bottom surface of the culture tank (31), and sends light irradiation emitting from the light irradiation member (38) itself to the algae.

8. The algae culturing apparatus (11) according to any one of claims 1 to 7,
    wherein the culture tank (31) is formed such that a base surface of the algae culturing apparatus (11) is dug into so as to be deeper than a ground level.

9. The algae culturing apparatus (11) according to any one of claims 1 to 8, further comprising a sheet (32) which covers the culture tank (31) and covers the inside wall part and the bottom portion of the culture tank (31),
    wherein the culture solution is accommodated in the culture tank (31) through the sheet (32).

10. The algae culturing apparatus (11) according to any one of claims 1 to 9,
    wherein a carbon dioxide supply port for supplying carbon dioxide to the culture solution accommodated in the culture tank (31) is provided to the culture tank (31).

11. The algae culturing apparatus (11) according to claim 10, wherein the carbon dioxide supply port is disposed at the center of the bottom surface of the culture tank (31).

12. The algae culturing apparatus (11) according to any one of claims 1 to 11, wherein an algae recovery port for recovering the algae cultured in the culture solution along with the culture solution is provided to the culture tank (31).

13. The algae culturing apparatus (11) according to claim 12, wherein the algae recovery port is disposed on the bottom surface of the culture tank (31).

14. The algae culturing apparatus (11) according to any one of claims 1 to 13, wherein a flow rate v of the culture solution on an outer peripheral edge in the culture tank (31) expressed by an equation (1) below is 12 m/min or more:

$$v = 2\pi \frac{\sqrt{\frac{\phi \cdot \text{Watt}}{D \cdot \pi}}}{t} \quad \cdots (1)$$

where, in the equation (1), $\varphi$ is a parameter which is obtained by dividing a volume of the culture solution by an output of the flow generator (33) required for the culture of the algae, Watt is an output of the flow generator (33) required for the culture of the algae, D is a depth of the culture solution which is accommodated in the culture tank (31) and has a numerical value within a range of 0.4 m to 1 m, and t is time which is required to allow the culture solution to complete one revolution around the outer peripheral edge in the culture tank (31).

15. The algae culturing apparatus (11) according to any one of claims 1 to 14, wherein one flow generator (33) is provided with respect to one culture tank (31).

16. An algae culturing system, comprising:

a plurality of the algae culturing apparatuses (11) according to any one of claims 1 to 15; a power storage portion (23) which is electrically connected to the plurality of algae culturing apparatuses (11); and a solar cell panel (21) which is electrically connected to the power storage portion (23), wherein electricity generated by the solar cell panel (21) is supplied to the power storage portion (23) so as to drive a plurality of the flow generators (33).

17. The algae culturing system according to claim 16, further comprising a carbon dioxide supply source (26) which supplies carbon dioxide to the culture solution accommodated in the culture tank (31) through the carbon dioxide supply port which is provided in the culture tank (31).

18. The algae culturing system according to claim 16 or 17, further comprising a solar light collection portion (24) which collects solar light and supplies the collected solar light to the light irradiation member (38) which is provided in the algae culturing apparatus (11).

19. The algae culturing system according to any one of claims 16 to 18, further comprising an algae recovery line (16, 71), a first end portion of which is connected to the algae recovery port provided in the culture tank (31) to recover the algae cultured in the culture solution along with the culture solution, wherein a second end portion of the algae recovery line (16, 71) is disposed at a position below the first end portion to recover the algae from the culture tank (31) utilizing height difference of both first and second end portions of the algae recovery line (16, 71).

20. The algae culturing system according to claim 19, wherein at least a portion of the algae recovery line (71) is inclined downward from the first end portion toward the second end portion.

21. The algae culturing system according to any one of claims 16 to 20, wherein the solar cell panel (21) is installed above the culture tank (31).

**Patentansprüche**

1. Algenkultivierungsvorrichtung (11), umfassend:

einen Kulturtank (31), der konfiguriert ist, um Algen und eine Kulturlösung (31) aufzunehmen, um die Algen zu kultivieren; und einen Flussgenerator (33), der auf dem Kulturtank (31) installiert und konfiguriert ist, um einen Fluss der Kulturlösung vom Zentrum des Kulturtanks (31) hin zu einem inneren Wandteil des Kulturtanks (31) zu einer flüssigen Oberfläche der Kulturlösung und in einer Region der Kulturlösung nahe der flüssigen Oberfläche durch Bewegung der Kulturlösung auf der flüssigen Oberfläche und in der Region nahe der flüssigen Oberfläche im Kulturtank (31) vom Zentrum des Kulturtanks (31) hin zum inneren Wandteil des Kulturtanks (31) zu erzeugen, wobei:
der Flussgenerator (33) Folgendes umfasst:

einen Motor (41);
eine Rotationswelle (43), von der ein Endabschnitt mit dem Motor (41) verbunden ist;
eine Vielzahl von Stützelementen (46), die radial um die Rotationswelle (43) angeordnet ist, wobei jedes der Vielzahl von Stützelementen (46) mit dem anderen Endabschnitt der Rotationswelle (43) auf einem unteren Endabschnitt davon verbunden ist; und
eine Vielzahl von Schaufelelementen (45), die an Spitzenabschnitte der Stützelemente (46) befestigt sind, und wobei
in einem Zustand, in dem die Rotationswelle (43) derart angeordnet ist, das sie orthogonal zur flüssigen Oberfläche der Kulturlösung ist, die im Kulturtank (31) aufgenommen ist, die Vielzahl von Schaufelelementen (45) derart angeordnet ist, dass sie näher am inneren Wandteil des Kulturtanks (31) als die Rotationswelle (43) ist, und von den Stützelementen (46) hängt, um mit der Kulturlösung in Kontakt zu kommen,
ein spezifisches Gewicht des Stützelements geringer als dasjenige der Kulturlösung ist und das Stützelement (46) ein schwimmendes Auftriebsmaterial ist, um den Auftrieb des Flussgenerators (33) mit Bezug auf die Kulturlösung sicherzustellen, und
die Algenkultivierungsvorrichtung (11) weiter ein Element zur Regulierung der Position des Flussgenerators (34) umfasst, das konfiguriert ist, um eine Position des Flussgenerators (33) am zentralen Abschnitt des Kulturtanks (31) in einen Zustand zu regeln, in dem der Flussgenerator (33) auf der Kulturlösung schwimmt.

2. Algenkultivierungsvorrichtung (11) nach Anspruch 1,
wobei die Vielzahl von Stützelementen (46) derart angeordnet ist, dass alle Winkel zwischen benachbarten Stützelementen (46) untereinander gleich sind.

3. Algenkultivierungsvorrichtung nach einem der Ansprüche 1 oder 2,
wobei im Kulturtank (31) der innere Wandteil eine zylindrische Form in einer Draufsicht aufweist und der Flussgenerator (33) am zentralen Abschnitt des Kulturtanks (31) angeordnet ist.

4. Algenkultivierungsvorrichtung nach einem der Ansprüche 1 bis 3,
wobei der Flussgenerator (33) konfiguriert ist, um den Fluss der Kulturlösung vom Zentrum des Kulturtanks (31) hin zum inneren Wandteil in alle Richtungen vom Zentrum des Kulturtanks (31) mit Bezug auf die Kulturlösung zu erzeugen.

5. Algenkultivierungsvorrichtung (11) nach einem der Ansprüche 1 bis 4, weiter umfassend ein Lichtstrahlungselement (38), das Lichtstrahlung an die Algen sendet, die in der Kulturlösung vorhanden sind, die im Kulturstank (31) aufgenommen ist.

6. Algenkultivierungsvorrichtung (11) nach Anspruch 5,
wobei das Lichtstrahlungselement (38) eine optischen Faser einschließt, die auf einer unteren Fläche des Kulturtanks (31) angeordnet ist, Licht von einer externen Lichtquelle in die unter Fläche des Kulturtanks (31) durch die optische Faser leitet und die Lichtstrahlung an die Algen sendet.

7. Algenkultivierungsvorrichtung nach Anspruch 5,
wobei das Lichtstrahlungselement (38) auf einer unteren Fläche des Kulturtanks (31) angeordnet ist und Lichtstrahlung, die vom Lichtstrahlungselement (38) selbst emittiert, an die Algen sendet.

8. Algenkultivierungsvorrichtung (11) nach einem der Ansprüche 1 bis 7,
wobei der Kulturtank (31) derart gebildet ist, dass eine untere Fläche der Algenkultivierungsvorrichtung (11) eingegraben ist, um tiefer als ein Bodenniveau zu sein.

9. Algenkultivierungsvorrichtung (11) nach einem der Ansprüche 1 bis 8, weiter umfassend ein Folie (32), die den Kulturtank (31) abdeckt und den inneren Wandteil und den unteren Abschnitt des Kulturtanks (31) abdeckt,
wobei die Kulturlösung im Kulturtank (31) durch die Folie aufgenommen ist.

10. Algenkultivierungsvorrichtung (11) nach einem der Ansprüche 1 bis 9,
wobei ein Kohlendioxid-Versorgungsanschluss zur Versorgung der Kulturlösung, die im Kulturtank (31) aufgenommen ist, mit Kohlendioxid dem Kulturtank (31) bereitgestellt ist.

11. Algenkultivierungsvorrichtung (11) nach Anspruch 10,
wobei ein Kohlendioxid-Versorgungsanschluss im Zentrum der unteren Fläche des Kulturtanks (31) angeordnet ist.

**12.** Algenkultivierungsvorrichtung (11) nach einem der Ansprüche 1 bis 11, wobei ein Algenwiedergewinnungsanschluss zur Wiedergewinnung der Algen, die in der Kulturlösung kultiviert wurden, zusammen mit der Kulturlösung dem Kulturtank (31) bereitgestellt ist.

**13.** Algenkultivierungsvorrichtung (11) nach Anspruch 12, wobei der Algenwiedergewinnungsanschluss auf der unteren Fläche des Kulturtanks (31) angeordnet ist.

**14.** Algenkultivierungsvorrichtung (11) nach einem der Ansprüche 1 bis 13, wobei eine Flussrate v der Kulturlösung auf einer äußeren Umfangskante im Kulturtank (31), exprimiert durch eine Gleichung (1) unten, 12 m/min oder mehr ist:

$$v = 2\pi \sqrt{\dfrac{\phi \cdot Watt}{\dfrac{D \cdot \pi}{t}}} \qquad \cdots (1),$$

wobei in der Gleichung (1) $\varphi$ ein Parameter ist, der durch Dividieren eines Volumens der Kulturlösung durch einen Ausgang des Flussgenerators (33), erforderlich für die Kultur der Algen, erhalten wird, Watt ein Ausgang des Flussgenerators (33), erforderlich für die Kultur der Algen, ist, D eine Tiefe der Kulturlösung ist, die im Kulturtank (31) aufgenommen ist und einen numerischen Wert innerhalb des Bereichs von 0,4 m bis 1 m aufweist, und t die Zeit ist, die erforderlich ist, um zu ermöglichen, dass die Kulturlösung eine Umdrehung um die äußere Umfangskante im Kulturtank (31) vervollständigt.

**15.** Algenkultivierungsvorrichtung (11) nach einem der Ansprüche 1 bis 14, wobei ein Flussgenerator (33) mit Bezug auf einen Kulturtank (31) bereitgestellt ist.

**16.** Algenkultivierungssystem, umfassend eine Vielzahl der Algenkultivierungsvorrichtungen (11) nach einem der Ansprüche 1 bis 15, einen Leistungsspeicherabschnitt (23), der elektrisch mit der Vielzahl von Algenkultivierungsvorrichtungen (11) verbunden ist; und ein Solarzellenpaneel (21), das elektrisch mit dem Leistungsspeicherabschnitt (23) verbunden ist, wobei der Strom, erzeugt durch das Solarzellenpaneel (21), an den Leistungsspeicherabschnitt (23) geliefert wird, um eine Vielzahl der Flussgeneratoren (33) anzutreiben.

**17.** Algenkultivierungssystem nach Anspruch 16, weiter umfassend eine Kohlendioxidversorgungsquelle (26), die Kohlendioxid an die Kulturlösung, die im Kulturtank (31) aufgenommen ist, durch den Kohlendioxid-Versorgungsanschluss liefert, der dem Kulturtank (31) bereitgestellt ist.

**18.** Algenkultivierungssystem nach Anspruch 16 oder 17, weiter umfassend einen Sonnenlichtsammelabschnitt (24), der Sonnenlicht sammelt und das gesammelte Sonnenlicht an das Lichtstrahlungselement (38) liefert, das in der Algenkultivierungsvorrichtung (11) bereitgestellt ist.

**19.** Algenkultivierungssystem nach einem der Ansprüche 16 bis 18, weiter umfassend eine Algenwiedergewinnungsleitung (16, 71), von der ein erster Endabschnitt, mit dem Algenwiedergewinnungsanschluss verbunden ist, der im Kulturtank (31) bereitgestellt ist, um die Algen, die in der Kulturlösung kultiviert wurden, zusammen mit der Kulturlösung wiederzugewinnen, wobei ein zweiter Endabschnitt der Algenwiedergewinnungsleitung (16, 71) an einer Position unter dem ersten Endabschnitt angeordnet ist, um die Algen aus dem Kulturtank (31) unter Verwendung der Höhendifferenz sowohl des ersten als auch des zweiten Endabschnitts der Algenwiedergewinnungsleitung (16, 71) wiederzugewinnen.

**20.** Algenkultivierungssystem nach Anspruch 19, wobei mindestens ein Abschnitt der Algenwiedergewinnungsleitung (71) vom ersten Endabschnitt hin zum zweiten Endabschnitt nach unten geneigt ist.

**21.** Algenkultivierungssystem nach einem der Ansprüche 16 bis 20, wobei das Solarzellenpaneel (21) über dem Kulturtank (31) installiert ist.

**Revendications**

**1.** Appareil de culture d'algues (11), comprenant :

une cuve de culture (31) conçue pour recevoir des algues et une solution de culture destinée à la culture des algues ; et
un générateur de flux (33) installé sur la cuve de culture (31) et conçu pour générer un flux de la solution de culture du centre de la cuve de culture (31) vers une partie de paroi intérieure de la cuve de culture (31) sur une surface liquide de la solution de culture et dans une région de la solution de culture proche de la surface liquide en déplaçant la solution de culture sur la surface liquide et dans la région proche de la surface liquide dans la cuve de culture (31) du centre de la cuve de culture (31) vers la partie de paroi intérieure de la cuve de culture (31), dans lequel :

le générateur de flux (33) inclut :

un moteur (41) ;

un arbre rotatif (43), dont une partie d'extrémité est relié au moteur (41) ;

une pluralité d'éléments de support (46) disposés radialement autour de l'arbre rotatif (43), chacun de la pluralité d'éléments de support (46) étant relié à l'autre partie d'extrémité de l'arbre rotatif (43) sur une partie d'extrémité de base de celui-ci ; et

une pluralité d'éléments formant pales (45) qui sont fixés aux parties de pointe des éléments de support (46), et dans lequel

dans un état où l'arbre rotatif (43) est disposé de sorte à être orthogonal à la surface liquide de la solution de culture reçue dans la cuve de culture (31), la pluralité d'éléments formant pales (45) sont disposés de sorte à être plus proches de la partie de paroi intérieure de la cuve de culture (31) que l'arbre rotatif (43), et sont suspendus aux éléments de support (46) pour venir en contact avec la solution de culture,

un poids spécifique de l'élément de support (46) est plus léger que celui de la solution de culture, et l'élément de support (46) est un matériau flottant destiné à garantir la flottabilité du générateur de flux (33) par rapport à la solution de culture, et

l'appareil de culture d'algues (11) comprend en outre un élément de régulation de position de générateur de flux (34) qui est conçu pour réguler une position du générateur de flux (33) au niveau de la partie centrale de la cuve de culture (31) dans un état où le générateur de flux (33) flotte sur la solution de culture.

2. Appareil de culture d'algues (11) selon la revendication 1,
dans lequel la pluralité d'éléments de support (46) sont disposés de telle sorte que tous les angles entre les éléments de support (46) adjacents sont identiques les uns aux autres.

3. Appareil de culture d'algues (11) selon la revendication 1 ou 2,
dans lequel dans la cuve de culture (31), la partie de paroi intérieure a une forme cylindrique sur une vue en plan, et le générateur de flux (33) est disposé au niveau de la partie centrale de la cuve de culture (31).

4. Appareil de culture d'algues (11) selon l'une quelconque des revendications 1 à 3,
dans lequel le générateur de flux (33) est conçu pour générer le flux de la solution de culture du centre de la cuve de culture (31) vers la partie de paroi intérieure dans toutes les directions depuis le centre de la cuve de culture (31) par rapport à la solution de culture.

5. Appareil de culture d'algues (11) selon l'une quelconque des revendications 1 à 4, comprenant en outre un élément d'irradiation de lumière (38) qui envoie une irradiation de lumière vers les algues présentes dans la solution de culture reçue dans la cuve de culture (31).

6. Appareil de culture d'algues (11) selon la revendication 5,
dans lequel l'élément d'irradiation de lumière (38) inclut une fibre optique qui est disposée sur une surface inférieure de la cuve de culture (31), guide la lumière d'une source de lumière externe dans la surface inférieure de la cuve de culture (31) à travers la fibre optique, et envoie l'irradiation de lumière vers les algues.

7. Appareil de culture d'algues (11) selon la revendication 5,
dans lequel l'élément d'irradiation de lumière (38) est disposé sur une surface inférieure de la cuve de culture (31), et envoie l'irradiation de lumière en émettant depuis l'élément d'irradiation de lumière (38) lui-même vers les algues.

8. Appareil de culture d'algues (11) selon l'une quelconque des revendications 1 à 7,
dans lequel la cuve de culture (31) est formée de telle sorte qu'une surface de base de l'appareil de culture d'algues (11) est creusée de sorte à être plus profonde qu'un niveau du sol.

9. Appareil de la culture d'algues (11) selon l'une quelconque des revendications 1 à 8, comprenant en outre une feuille (32) qui couvre le réservoir de culture (31) et couvre la partie de paroi intérieure et la partie inférieure du réservoir de culture (31),
dans lequel la solution de culture est reçue dans la cuve de culture (31) à travers la feuille (32).

10. Appareil de culture d'algues (11) selon l'une quelconque des revendications 1 à 9,
dans lequel un orifice d'alimentation en dioxyde de carbone destiné à fournir du dioxyde de carbone à la solution de culture reçue dans la cuve de culture (31) est prévu sur la cuve de culture (31)

11. Appareil de culture d'algues (11) selon la revendication 10,
dans lequel l'orifice d'alimentation en dioxyde de carbone est disposé au centre de la surface inférieure de la cuve de culture (31).

**12.** Appareil de culture d'algues (11) selon l'une quelconque des revendications 1 à 11,
dans lequel un orifice de récupération d'algues destiné à récupérer les algues cultivées dans la solution de culture ainsi que la solution de culture est prévu sur la cuve de culture (31).

**13.** Appareil de culture d'algues (11) selon la revendication 12,
dans lequel l'orifice de récupération d'algues est disposé sur la surface inférieure de la cuve de culture (31).

**14.** Appareil de culture d'algues (11) selon l'une quelconque des revendications 1 à 13,
dans lequel un débit v de la solution de culture sur un bord périphérique externe dans la cuve de culture (31) exprimé par une équation (1) ci-dessous est de 12 m/min. ou plus :

$$v = 2\pi \frac{\sqrt{\dfrac{\phi.Watt}{D.\pi}}}{t} \qquad \dots(1)$$

où, dans l'équation (1), φ est un paramètre qui est obtenu en divisant un volume de la solution de culture par une puissance du générateur de flux (33) nécessaire pour la culture des algues, Watt est une puissance du générateur de flux (33) nécessaire pour la culture des algues, D est une profondeur de la solution de culture qui est reçue dans la cuve de culture (31) et a une valeur numérique dans une plage de 0,4 m à 1 m, et t est une durée qui est nécessaire pour permettre à la solution de culture de terminer un tour autour du bord périphérique externe dans la cuve de culture (31).

**15.** Appareil de culture d'algues (11) selon l'une quelconque des revendications 1 à 14, dans lequel un générateur de flux (33) est placé par rapport à une cuve de culture (31).

**16.** Système de culture d'algues, comprenant :

une pluralité des appareils de culture d'algues (11) selon l'une quelconque des revendications 1 à 15 ;
une partie de stockage d'énergie (23) qui est connectée électriquement à la pluralité d'appareils de culture d'algues (11) ; et
un panneau de cellules solaires (21) qui est connecté électriquement à la partie de stockage d'énergie (23),
dans lequel l'électricité générée par le panneau de cellules solaires (21) est fournie à la partie de stockage d'énergie (23) de sorte à entraîner une pluralité de générateurs de flux (33).

**17.** Système de culture d'algues selon la revendication 16, comprenant en outre une source d'alimentation en dioxyde de carbone (26) qui fournit du dioxyde de carbone à la solution de culture reçue dans la cuve de culture (31) par l'orifice d'alimentation en dioxyde de carbone qui est prévu dans la cuve de culture (31).

**18.** Système de culture d'algues selon la revendication 16 ou 17, comprenant en outre une partie de collecte de lumière solaire (24) qui collecte de la lumière solaire et fournit la lumière solaire collectée à l'élément d'irradiation de lumière (38) qui est prévu dans l'appareil de culture d'algues (11).

**19.** Système de culture d'algues selon l'une quelconque des revendications 16 à 18, comprenant en outre une conduite de récupération d'algues, (16, 71), dont une première partie d'extrémité est reliée à l'orifice de récupération d'algues prévu dans la cuve de culture (31) pour récupérer les algues cultivées dans la solution de culture ainsi que la solution de culture, dans lequel une seconde partie d'extrémité de la conduite de récupération d'algues (16, 71) est disposée dans une position en dessous de la première partie d'extrémité pour récupérer les algues de la cuve de culture (31) en utilisant la différence de hauteur des première et seconde parties d'extrémité à la fois de la conduite de récupération d'algues (16, 71).

**20.** Système de culture d'algues selon la revendication 19,
dans lequel au moins une partie de la conduite de récupération d'algues (71) est inclinée vers le bas de la première partie d'extrémité vers la seconde partie d'extrémité.

**21.** Système de culture d'algues selon l'une quelconque des revendications 16 à 20,
dans lequel le panneau de cellules solaires (21) est installé au-dessus de la cuve de culture (31).

FIG. 1

EP 3 081 628 B1

FIG. 2

EP 3 081 628 B1

FIG. 3

EP 3 081 628 B1

FIG. 4

EP 3 081 628 B1

# FIG. 5

EP 3 081 628 B1

## FIG. 6

## FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012077081 A1
- US 2013102076 A1
- JP 2011254724 A
- JP H07000176 A